# EUROPÄISCHE PATENTANMELDUNG

(11) **EP 0 524 639 A2**
(43) Veröffentlichungstag der Anmeldung: **27.01.1993**
(21) Anmeldenummer: 92112689.2
(22) Anmeldetag: 24.07.1992
(51) Int. Cl.: C07B 59/00, A61K 49/02, C07D 257/02, C07F 13/00

(54) **Verfahren zur Markierung von Substanzen mit Technetium oder Rhenium**

(30) Priorität: 26.07.1991 DE 4124789
(71) Anmelder: HOECHST AKTIENGESELLSCHAFT, D-65926 Frankfurt am Main (DE)
(72) Erfinder: Bremer, Karlheinz, Dr., W-6232 Bad Soden am Taunus (DE); Hachmann, Henning, Dr., W-6000 Frankfurt am Main (DE); Kuhlmann, Ludwig, Dr., W-6093 Flörsheim am Main (DE); Magerstädt, Michael, Dr., W-6073 Egelsbach (DE); Schwarz, Alexander, Dr., W-6093 Flörsheim am Main (DE); Steinsträsser, Axel, Dr., W-6237 Liederbach (DE); Walch, Axel, Dr., W-6000 Frankfurt am Main (DE); Wiesner, Matthias, Dr., W-6500 Mainz (DE)

(57) **Zusammenfassung**

Die Erfindung betrifft ein Verfahren zur Markierung von Substanzen mit Technetium- und/oder Rheniumisotopen mit einem makrocyclischen Komplexbildner der Formel I, indem man die zu markierende Substanz mit dem Komplexbildner versetzt und die so erhaltene Verbindung markiert, oder zunächst den Komplexbildner markiert und anschließend die so erhaltene Verbindung mit der zu markierenden Verbindung umsetzt.

## Beschreibung

Gegenstand der Erfindung ist ein Verfahren zur Markierung von Substanzen mit - insbesondere radioaktiven - Technetium- bzw. Rheniumisotopen mit Hilfe von Komplexbildnern, sowie die Verwendung dieser markierten Substanzen.

Die Anwendungsmöglichkeiten von Radionukliden in der Technik sind sehr vielfältig. Sie erstrecken sich beispielsweise auf die Prüfung einer Durchmischung, auf die verdünnungsanalytische Bestimmung von Mengen oder Volumina, auf die Messung der Strömungsgeschwindigkeit und Aufnahme der Verweilzeit an kontinuierlich arbeitenden Produktionsanlagen.

Meist genügt jedoch nicht die bloße Beimischung eines radioaktiven Nuklids, sondern beispielsweise bei der "Verfolgung" einer bestimmten Komponente eines Systems, muß das radioaktive Nuklid physikalisch oder besser noch chemisch an mindestens eine Verbindung der zu untersuchenden Komponente gekoppelt werden und dies nach Möglichkeit ohne die physikalischen und chemischen Eigenschaften der betreffenden Verbindung zu beeinflussen.

Die Immunszintigraphie, eine nicht-invasive Methode zur Darstellung von pathologischen Veränderungen am Patienten durch Injektion radioaktiv markierter Antikörper, bzw. deren Fragmente oder gentechnologisch hergestellter immunreaktiver Proteine, gewinnt mehr und mehr klinische Bedeutung, insbesondere in der Diagnose maligner Erkrankungen.

Insbesondere Technetium-99m ist wegen seiner günstigen physikalischen Eigenschaften (Fehlen einer Korpuskularstrahlung, γ-Energie von 140 keV und Halbwertszeit von 6 Stunden) und der damit verbundenen geringen Strahlenbelastung zum wichtigsten Radionuklid in der nuklearmedizinischen Diagnostika geworden.

Die Rheniumisotope Re-186 und Re-188 sind β-emittierende Nuklide. Diese können zur Tumortherapie eingesetzt werden, da die energiereiche β-Strahlung cytotoxisch wirkt. Eine selektive Anreicherung im Tumor kann ebenfalls durch Konjugation an z.B. tumorspezifische monoklonale Antikörper erfolgen. Die Redox- und Koordinationschemie des Rheniums ist der des Technetiums sehr ähnlich.

Technetium-99m, das sich aus Nuklidgeneratoren gewinnen läßt, liegt zunächst als Pertechnetat vor, das in dieser Form, z.B. für die Schilddrüsen- und Hirnszintigraphie geeignet ist. Die Szintigraphie anderer Organe mittels Technetium-99m gelingt mit Hilfe bestimmter "Transportsubstanzen", die einerseits in der Lage sind Technetium zu binden, und andererseits das Radionuklid im Zielorgan mit hoher Selektivität anzureichern. Zur Markierung der organspezifischen "Transportsubstanz" mit Technetium-99m muß das aus dem Nuklidgenerator eluierte Pertechnetat zuerst in eine niedrigere Oxidationsstufe überführt werden. In dieser reduzierten Form bildet Technetium mit der organspezifischen Substanz mehr oder weniger stabile Verbindungen. Für die Knochenszintigraphie werden z.B. Tc-99m-Phosphorsäure-Derivate, vor allem organische Phosphonsäuren, eingesetzt. So liegt in der EP-A 0 002 485 beschriebenen Markierungseinheit das Natriumsalz der 3,3-Diphosphono-1,2-propandicarbonsäure als organspezifische "Transportsubstanz" vor. In der EP-A 0 108 253 werden Tc-99m-Tri- und Tetraphosphonsäuren zur szintigraphischen Darstellung des RES, insbesondere der Leber, beschrieben. Der Tc-99m-Komplex mit Diethylentriaminpentaessigsäure (DTPA) findet zur Diagnostik von Nierenerkrankungen bzw. pathologischen Hirnprozessen Anwendung.

Zur Markierung bestimmter Substanzen mit Technetium-99m und zur Herstellung von für den klinischen Routinebedarf geeigneten Testkits wurden spezielle Verfahren entwickelt und beschrieben. Zur Präparierung von Markierungsbestecken für biologisch interessante Makromoleküle, insbesondere Porphyrine, Dextrane, Cytochrome und Myoglobin, wird eine Methode beschrieben (G.D. Zanelli, D. Ellison, M.P. Barrowcliffe, Nucl. Med. Commun. 8, 199-206, 1987), bei der die zu markierende Substanz zusammen mit p-Aminobenzoesäure und einer salzsauren SnCl₂-Lösung lyophylisiert wird. Zur Rekonstitution und Markierung dieses Kits gibt man Tc-99m-Generatoreluat, das vorher mit genügend Pufferlösung, z.B. Citrat-Natriumchlorid-Puffer pH 9,5, verdünnt wurde, hinzu. Für säureempfindliche Substanzen ist diese Methode jedoch nicht geeignet.

Bei einem anderen Verfahren (E.K.J. Pauwels, R.I.J. Feitsma, WO 86/03010) wird durch vierstündiges Erhitzen in stark salzsaurer Lösung auf 140°C Tc-99m-Pertechnetat zuerst reduziert und an einer Verbindung, die eine Aminogruppe enthält, z.B. Dimethylformamid, gebunden. Das reaktionsfähige Tc-99m-markierte Zwischenprodukt, das als schwerlösliche kristalline Substanz ausfällt, wird in einer Pufferlösung, z.B. Natriumcarbonatlösung, durch einstündige Inkubation bei Raumtemperatur mit der zu markierenden Verbindung umgesetzt. Die Methode arbeitet zwar zinnfrei, ist aber wegen der aufwendigen Verfahrensschritte für die Routineanwendung kaum geeignet.

Zur Markierung von Proteinen, insbesondere Antikörpern, kennt man zwei verschiedene Wege. Bei der direkten Methode wird das reduzierte Technetium-99m durch Donorgruppen (Amino-, Amid-, Thiol- etc.) des Proteins gebunden.

Solche Verfahren sind in der EP-A 0 005 638 und der US-A 4.478.815 beschrieben. Dort werden Zinn-II-Salze im Überschuß zur gleichzeitigen reduktiven Spaltung von Disulfid-Brücken und zur Reduktion des zugesetzten Tc-99m-Pertechnetats benutzt. Im allgemeinen benötigt man zur Spaltung der -S-S-Bindung längere Inkubationszeiten (24 Stunden), wobei F(ab')₂-Fragmente partiell zu F(ab)-Fragmenten gespalten werden. Neuere Literaturangaben (z.B. Journal of Nuclear Medicine 27 (1986), Seite 685-93 und 1315-20 sowie International Journal of Nuclear Medicine Biology 12 (1985) Seiten 3-8) zeigen, daß das Verhältnis der beiden Fragmente abhängig istvon der "Bezinnungsreaktion" und daß sich das Verhältnis der beiden Komponenten nach der Tc-99m-Markierung nicht mehr nennenswert ändert, wobei die Hauptkomponente Tc-99m-markiertes F(ab') ist. In allen Fällen mußte das markierte F(ab')-Fragment nachgereinigt werden, da trotz mindestens 30-minütiger Reaktionszeit keine quantitative Umsetzung des Pertechnetats erreicht wurde.

Bei einer schnellen chemischen Methode zur Tc-99m-Markierung humaner Plasmaproteine (D.W. Wong, F. Mishkin, T. Lee, J. Nucl. Med. 20, 967-72, 1979) wird Pertechnetat zuerst in saurer Lösung durch Zinn-II-Ionen reduziert und das reduzierte Technetium dann mit Protein umgesetzt.

Unter Zuhilfenahme von bifunktionalen Komplexbildnern läßt sich eine stabile Markierung von Substanzen mit Radioisotopen erreichen.

In dem US-Patent 4.479.930 werden die cyclischen Anhydride von DTPA und EDTA als Chelatisierungsmittel nicht nur für In-111 und Ga-67, sondern auch für Tc-99m angeführt. In der EP-A 0 035 765 wird die Verwendung von Deferroxamin als Komplexierungsagens für Technetium-99m an Proteine erwähnt. In der internationalen Patentanmeldung WO 85/3063 werden die partiell reduzierten Disulfid-Brücken im Antikörper mit dem Natriumsalz des Tetrachlornitridotechnetats, das durch Reaktion von Pertechnetat mit Natriumazid vorher präpariert werden muß, umgesetzt. In der EP-A 0 194 853 benutzt man ebenfalls durch Reduktion in Antikörperfragmenten erzeugte freie Thiolgruppen zur Bindung von [(7-Maleimidoheptyl)imino-bis(ethylennitrilo)]-tetraessigsäure als Chelatkomplex. Die Kupplung des Komplexes an den Antikörper erfolgt über die Reaktion der SH-Gruppen mit der Doppelbindung im Maleinimidteil der Komplexverbindung, während das radioaktive Metallion über die Nitrilodiessigsäure-Reste komplexiert wird.

Um Technetium-99m diagnostisch breit nutzen zu können, ist es notwendig, dieses Nuklid in das zu untersuchende Organ selektiv zu transportieren. Aus anderen Organen oder Organsystemen sollte das Technetium-99m wieder schnell eliminiert oder überhaupt nicht eingebracht werden, um jede unnötige Strahlenbelastung für den Patienten zu vermeiden. Zu diesem Zweck werden bisher vorwiegend Substanzen eingesetzt, die direkt mit Technetium-99m markierbar sind und eine hohe Organspezifität aufweisen. Darüber hinaus gibt es jedoch eine Reihe von Substanzen, die zwar eine hohe Organspezifität aufweisen, aber nicht direkt markierbar sind. Dies können Proteine (Fibrinogen, Humanserumalbumin), Enzyme (Streptokinase, Lactatdehydrogenase), Zucker (Dextran, Glucose) oder auch Polymere sein. Dazu gehören ebenfalls niedermolekulare Substanzen wie etwa Fettsäuren, die durch den hohen Energiebedarf des Herzens sich im Myocardgewebe anreichern. Um diese Substanzen markieren zu können, werden sie mit Komplexbildners gekoppelt, die ihrerseits Technetium-99m fest binden können.

Als geeignete Komplexbildner für die Komplexierung von Technetium- und Rheniumisotopen sind makrozyklische Amine, u.a. auch Cyclame, bekannt. Die Komplexierungsausbeute liegt für den Technetium-Cyclam Komplex bei 99 %, unter geeigneten Bedingungen. Einzelheiten über Technetium-Aminkomplexe sind in D.E. Troutner, J. Simon, A.R. Ketring, W.A. Volkert, R.A. Holmes, J. Nucl. Med. 21 (1980), 443 oder S.A. Zuckman, G.M. Freeman, D.E. Troutner, W.A. Volkert, R.A. Holmes, D.G. van der Keer, E.K. Barefield, Inorg. Ch. 20 (1981), 3386 oder J. Simon, D. Troutner, W.A. Volkert, R.A. Holmes, Radiochem. Radioanal. Lett. 47 (1981), 111 erwähnt. Auch substituierte Cyclame sind sowohl am 1-Stickstoff, als auch am 6-Kohlenstoff substituiert, bekannt (A.R. Ketring, D.E. Troutner et al., Int. J. Nucl. Med. Biol. 11 (1984), 113 oder J. Simon. Diss. Abstr. Int. B42 (1981), 645 oder M. Struden, T.A. Kaden, Helv. Chim. Acta 69 (1986), 2081 oder E. Kumura, R. Machida, M. Kodama, J. Am. Chem. Soc. 106 (1984), 5497).

Alle bisherigen Versuche, Amin- und auch andere Liganden an Proteine zu konjugieren (s. Fritzberg et al., J. Nucl. Med 27 (1986), 957 oder Tolman et al., J. Nucl. Med. 25 (1984), 20 oder Arano et al., Int. J. Nucl. Med. Biol. 12 (1986) 425) führten zu Produkten, die die hohen in vivo Stabilitätsansprüche nicht oder nur teilweise erfüllten.

In der EP-A-0 304 780 werden spezielle N- und C-substituierte makrocyclische Aminderivate vorgestellt, die eine verbesserte Stabilität aufweisen. Ihr Nachteil besteht aber darin, daß gerade in einem pH-Bereich, der für die zu markierende Substanz (z.B. Protein) unschädlich ist, die Stabilität des Komplexes nicht hinreichend groß ist. Lediglich im stark basischen pH-Bereich kann eine vollständige Komplexierung auch mit makrocyclischen Chelatbildnern erreicht werden.

Es war deshalb die Aufgabe der vorliegenden Erfindung, ein Verfahren zur Markierung von Substanzen mit Technetium oder Rhenium bereitzustellen, das im neutralen pH-Bereich, d.h. bei einem pH-Wert unterhalb 9, ein hohes Ausmaß der Komplexbildung ermöglicht. Es war weiterhin die Aufgabe der Erfindung, einen Chelatbildnerer für Technetium oder Rhenium bereitzustellen, der unter in vivo-Bedingungen, d.h. im neutralen pH-Bereich, eine hohe Stabilität aufweist.

Die Erfindung wird gelöst durch ein Verfahren zur Markierung von Substanzen mit Technetium- und/oder Rheniumisotopen, dadurch gekennzeichnet, daß
a) die zu markierende Substanz mit einem makrocyclischen Komplexbildner der allgemeinen Formel I 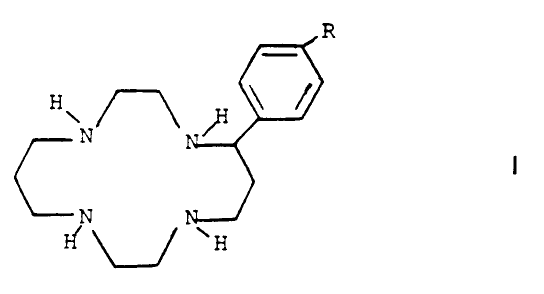 , worin
   - R: 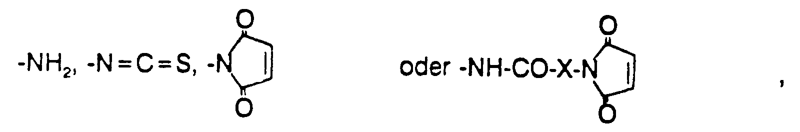 und
   - X: (C₁-C₆)-Alkylen, (C₆-C₁₂)-Arylen, (C₁-C₆)-Alk-(C₆-C₁₂)-arylen, insbesondere (C₁-bedeutet,
   umgesetzt wird und die so erhaltene Verbindung mit Tc-99, Tc-99m, Re-186 oder Re-188 markiert wird, indem man sie bei einem pH-Wert von unter 9 mit Pertechnetat-99, -99m oder Perrhenat-186, -188 und einem Reduktionsmittel versetzt oder daß man
b) zunächst den Tc-99-, Tc-99m-, Re-186- oder Re-188-Komplex durch Umsetzung einer Verbindung der Formel I mit der entsprechenden Per-Verbindung und einem Reduktionsmittel bei einem pH-Wert von unter 9 herstellt und anschließend diesen Komplex mit der zu markierenden Substanz umsetzt.

Die Bindung des Chelatbildners gemäß Formel I bzw. des Komplexes an die zu markierende Verbindung erfolgt über dem Rest R. Es handelt sich dabei um die Ausbildung einer kovalenten Bindung.

Die Verbindungen der allgemeinen Formel I werden von der EP-A-0 304 780 zwar umfaßt; ihre besondere Fähigkeit zur Ausbildung stabiler Komplexe bei neutralen pH-Wert war aber seinerzeit noch nicht bekannt. Gemäß Beispiel 4 werden trotz Komplexbildung bei pH 11 nur 10 % des eingesetzten Tc-99m gebunden.

Die erfindungsgemäß ausgewählten Verbindungen der Formel I weisen aber schon im neutralen Medium hohe Komplexstabilitäten auf: Mehr als 90 % der vorgelegten Tc- oder Re-Isotopen werden gebunden, vorzugsweise mehr als 95 %. Der pH-Wert soll daher unter 9, vorzugsweise unter 8 liegen (und über pH 6,5).

Von Cyclam (1,4,8,11-Tetraazacyclotetradecan) ist bekannt, daß eine vollständige Komplexierung von Tc-99m nur unter stark basischen Bedingungen, d.h. in einem für z.B. Antikörper ungeeigneten pH-Bereich, möglich ist. Da ein diagnostischer Testkit aber nur dann in klinisch handhabbarer Form vorliegt, wenn der Komplexbildner bereits an die targetspezifischen Proteine (z.B. den Antikörpern bzw. deren Fragmenten oder gentechnologisch hergestellten immunreaktiven Proteinen) gebunden in lyophilisierter Form vorliegt, so daß in der Klinik nur noch das Radionuklid (und ggf. das Reduktionsmittel) zugegeben werden muß, war es erstrebenswert, daß die Komplexierung in einem für die markierte Substanz unschädlichen pH-Bereich erfolgen kann. Monoklonale Antikörper überstehen z.B. eine Komplexierung bei pH 8 ohne signifikante Verringerung der Immunoreaktivität und Affinität.

Die im erfindungsgemäßen Verfahren angewendeten Verbindungen ermöglichen erstmals eine solche Handhabung. Im Hinblick auf die geringere Stabilität der aus der EP-A-0 304 780 bekannten Verbindungen gerade im basischen pH-Bereich, war es völlig überraschend, daß die oben genannten und speziell ausgewählten Verbindungen diese Wirkung gerade im für eine hohe Stabilität noch kritischeren neutralen pH-Bereich zeigen.

Bei dem erfindungsgemäßen Verfahren wird eine Verbindung gemäß Formel I mit Hilfe der funktionellen Gruppe R an die zu markierende Substanz gebunden. Gegebenenfalls nach entsprechender Reinigung des Konjugats (z.B. durch Ultrafiltration oder Dialyse bei Proteinen oder Polymeren, durch Säulenchromatographie bei niedermolekularen Substanzen wie Steroiden oder Lipiden) werden Technetium-99m in der Form von Pertechnetat bzw. Rhenium-186 oder -188 in der Form von Perrhenat und ein geeignetes Reduktionsmittel zur Reduktion des Pertechnetats bzw. Perrhenats in die für die Komplexierung benötigte Oxidationsstufe, in beliebiger Reihenfolge oder gemeinsam zugegeben. Das markierte Substrat wird gegebenenfalls nochmals gereinigt.

Alternativ kann auch zunächst der Technetium- bzw. Rheniumkomplex der Verbindung gemäß Formel I erzeugt werden und dann dieser mit der Substanz zum Konjugat umgesetzt werden. Hierbei verläuft die Komplexierung und Reduktion wie oben beschrieben. Die Komplexierungsreaktion wird im neutralen pH-Bereich durchgeführt. Insbesondere sind pH-Werte kleiner als 9, bevorzugt von kleiner als 8 bevorzugt (und über pH 6,5).

Die Herstellung von Verbindungen des Typs der allgemeinen Formel I ist aus der EP-A-0 304 780 bekannt.

Die Reduktion des Pertechnetats bzw. des Perrhenats kann nach literaturbekannten Verfahren erfolgen, bevorzugt mit einer Zinn-II-Verbindung. Besonders bevorzugt erfolgt die Reduktion mit einem komplexstabilisierten Zinn-II-Salz, nach einem "Markierungsverfahren", wie dies in der EP-A 0 304 780 vorgeschlagen wurde. Dabei wird zunächst die Zinn-II-Verbindung mit einem Komplexbildner, vorzugsweise einer Phosphorverbindung wie einem Phosphonat oder Pyrophosphat versetzt, der dafür sorgt, daß die Zinnverbindung vor allem im physiologischen pH-Bereich in Lösung bleibt. Diese komplexstabilisierte Zinn-II-Salz-Lösung kann dann entweder zu der zu markierenden Substanz gegeben werden, worauf die Zugabe der Pertechnetat- bzw. Perrhenat-Lösung erfolgt oder aber zu einer Mischung aus der zu markierenden Substanz und der Pertechnetat- bzw. Perrhenat-Lösung.

Als zu markierende Substanzen (Trägersubstanzen bzw. organspezifische Substanzen) kommen beispielsweise Proteine (-NH-, -NH₂- oder COO-Gruppen), Enzyme (-NH₂-, -OH-, -P=O-Gruppen), Zucker (-OH-Gruppen) Fettsäuren, Hormone oder Polymere in Frage. Insbesondere bevorzugt ist die Markierung von Antikörpern oder deren Fragmenten, besonders bevorzugt die von monoklonalen Antikörper(fragmenten) (z.B. F(ab')₂- oder F(ab')-Fragmenten) oder gentechnologisch hergestellte immunreaktive Proteine.

Die Erfindung betrifft weiterhin einen Testkit zum Nachweis von Tumoren, bestehend aus zwei getrennten, lyophilisierten Komponenten, von denen z.B. eine einen Antikörper, der gegen Tumor-assoziierte Antigene gerichtet ist, oder dessen F(ab')₂-Fragment im Gemisch mit einem Puffer enthält, wobei der Antikörper oder dessen Fragment mit einem makrocyclischen gemäß Formel I verknüpft ist, und die andere ein Reduktionsmittel enthält, das zur Reduktion und Bindung des Technetiums oder Rheniums an der Antikörperkomponente benötigt wird.

Im folgenden soll die Erfindung anhand von Beispielen erläutert werden:
1) Darstellung von 1-(p-Isothiocyanatophenyl)-2,5,9,12-tetraazacyclotetradecanhydrochlorid (R = -N=C=S)
1a) Synthese von 2,5,9,12-Tetraaza-13-(p-nitrophenyl)-cyclotetradecanon 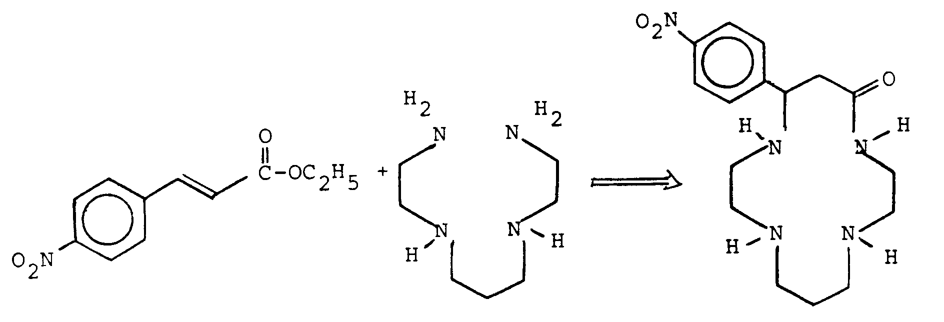 25 g 1,4,8,11-Tetraazaundecan (Fa. Strem, Kehl, Deutschland) und 34,5 g p-Nitrozimtsäureethylester (Fa. Aldrich, Steinheim, Deutschland) werden unter Feuchtigkeitsausschluß in 3 l wasserfreiem Methanol gelöst und über Siedesteinen 4 Wochen lang jeweils tagsüber 8 h rückflußgekocht und nachts bei Raumtemperatur stehen gelassen. Das Lösungsmittel wird im Vakuum abdestilliert. Der Rückstand wird mit 400 ml H₂O verrührt, filtriert und mit H₂O gewaschen.
   Die vereinigten Filtrate werden 1 x mit 200 ml Diethylether, dann 5 x mit 150 ml Methylenchlorid extrahiert. Die organischen Phasen werden über Na₂SO₄ getrocknet und zur Trockne eingeengt.
   Der so erhaltene Feststoff (9,2 g) wird auf einer Säule aus Kieselgel 60, welche vor Benutzung gründlich mit Methanol gespült wurde chromatographiert. Als Eluat wird zunächst Methanol, später ein Gradient von Methanol zu einem Methanol:konz. Ammoniak-Verhältnis von 9:1 eingesetzt. Fraktionen werden gesammelt und Dünnschichtchromatographie von diesen durchgeführt.
   Das Produkt läuft auf Kieselgelplatten im Methanol-konz. Ammoniak-Gemisch von 8:3 bei Rf = 0,7.
   Die entsprechenden Fraktionen werden eingeengt und das Produkt sorgfältig vakuumgetrocknet, um Methanolreste zu entfernen. Zur Entfernung von Kieselgelresten kann es aus wäßrigen oder alkoholischen Lösungen umkristallisiert werden.
   Das Produkt fällt lyophilisiert als fast farbloser, meist leicht gelblicher flockiger Feststoff an. Die Ausbeute beträgt 4,2 g. Meist kann aus den anderen Fraktionen der Säulenchromatographie durch wiederholte Chromatographie mehr Produkt isoliert werden. Die Charakterisierung erfolgt über ¹H-NMR, Massenspektrum, DC und IR.
1b) Reduktion von 2,5,9,12-Tetraaza-13-(p-nitrophenyl)-cyclotetradecanon zu 2,5,9,12-Tetraaza-13-(p-Aminophenyl)-cyclotetradecanon 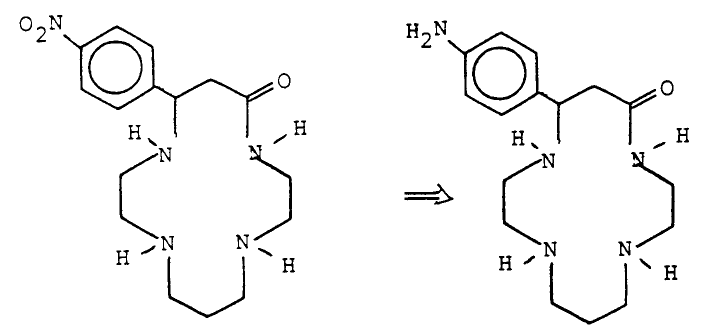 3 g 2,5,9,12-Tetraaza-13-(p-nitrophenyl)-cyclotetradecanon werden in 40 ml trockenem Methanol unter Luftausschluß gelöst. Dazu werden 200 mg Pd, verteilt auf Kohlenstoff, 5 % (Fa. Aldrich, Steinheim, Deutschland) gegeben. Nachdem die Apparatur gründlichst mit Stickstoff gespült wurde, wird unter starkem Rühren und Luftausschluß ein leichter H₂-Überdruck angelegt. Insgesamt (incl. Katalysatorsättigung) werden 900 ml Wasserstoff aufgenommen. Nach beendeter Reaktion wird mehrfach über Filtrierhilfsmittel (z.B. Corlite^{R}) filtriert, das Filtrat zur Trockne eingeengt und vakuumgetrocknet.
   Meist fällt das Produkt so rein an, daß keine weitere Reinigung erforderlich ist; bei verunreinigtem Produkt kann eine Säulenchromatographie analog der unter 1a) beschriebenen folgen; das Produkt läuft im DC auf Kieselgel in 8:3 Methanol:konz. Ammoniak bei Rf = 0,55. Ausbeute > 98 % d. Th., Charakterisierung über ¹H-NMR, DC, MS und IR.
1c) Reduktion von 2,5,9,12-Tetraaza-13-(p-Aminophenyl)-cyclotetradecanon zu 1-(p-Aminophenyl)-2,5,9,12-tetraazacyclotetradecan (R = NH₂) 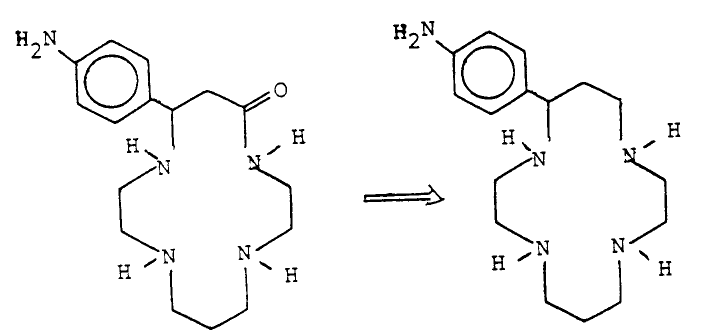 Die Reduktion der Lactamgruppierung kann nach gängigen Amidreduktionsverfahren mit Hydriden erfolgen. Kombinationen verschiedener Hydride zeigen die besten Ergebnisse. Beispielhaft ist die Reduktion mit Boran-THF beschrieben; auch LiBH₄, Lawesson's Reagenz und NaBH₄ sowie andere, dem Fachmann bekannte Reagenzien können eingesetzt werden. Die Ausbeute bei dieser Reaktion ist generell nicht quantitativ; das nicht reduzierte Lactam kann aber chromatographisch wiedergewonnen werden.
   1 g 2,5,9,12-Tetraaza-13-(p-Aminophenyl)-cyclotetradecanon wird in 60 ml über Natrium getrocknetem Tetrahydrofuran (THF) suspendiert (teilweise gelöst) und, nach Zugabe von 8 ml 1N Lösung von BH₃·THF, 2 Wochen lang rückflußgekocht. Der Boranüberschuß wird mit Methanol zerstört und die Lösung eingeengt. Nach 3-maligem Lösen in Methanol und jeweiligem Einengen des Lösungsmittels wird der Rückstand in trockenem Ethanol aufgenommen und HCl-Gas unter Feuchtigkeitsausschluß eingeleitet. Nach 1 h Einleitung (Gefäß im Eisbad gekühlt) wird 10 h lang rückflußgekocht, dann wieder eisgekühlt und erneut mit HCl-Gas gesättigt. Das Reaktionsgefäß wird verschlossen und über mehr als 10 h bei -18°C gekühlt. Der Niederschlag wird abfiltriert, mit trockenem Ethanol gewaschen und gefriergetrocknet. Aus der Mutterlauge kann weiteres Rohprodukt gewonnen werden.
   Eine Säulenchromatographie - wie unter 1a) beschrieben - wird mit dem vorher mit Ammoniak neutralisierten Rohprodukt durchgeführt. Das Produkt bleibt dabei auf der Säule und wird nach Elution des Ausgangsmaterials durch Erhöhung der Ammoniakkonzentration auf Methanol:konz. Ammoniak 1:1 eluiert. Es läuft im Dünnschichtchromatogramm bei Rf = 0,1 (Kieselgel, Methanol: konz. Ammoniak 8:3). Meist sind mehrere Säulenchromatographien hintereinander erforderlich, um das Produkt rein zu erhalten.
   Das Produkt wird durch ¹H-NMR (deutliche Verschiebung des 14-CH₂-Signals sowie des 1- (vorher 13-) CH-Signals und IR charakterisiert. Ausbeute: 100 mg (variiert mit der Reduktionsmethode). Aus den entsprechenden Fraktionen der Säulenchromatographie kann die Ausgangsverbindung in hoher Reinheit wiedergewonnen werden.
1d) Umsetzung von 1-(p-Aminophenyl)-2,5,9,12-tetraazacyclotetradecan (II, R = NH₂) mit Thiophosgen zu 1-(p-Isothiocyanatophenyl)-2,5,9,12-tetraazacyclotetradecanhydrochlorid (R = -N=C=S) 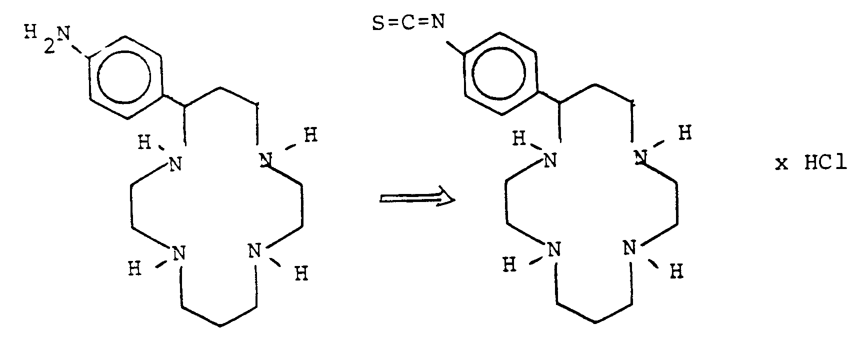 20 mg des Produkts von Beispiel 1c werden in 5 ml trockenem CH₂Cl₂ teilweise gelöst und unter Feuchtigkeitsausschluß mit 9 µl Thiophosgen und 100 mg festem Na₂CO₃ versetzt. Nach 4 h Rühren bei Raumtemperatur unter Feuchtigkeitsausschluß wird filtriert und das Filtrat im Vakuum getrocknet. Man erhält eine Verbindung gemäß Formel I, worin R = -N=C=S bedeutet.
   Das Produkt wird über ¹H-NMR (Zusammenfallen des Aromaten-Doppeldoubletts, Verbreiterung der aliphatischen Signale), IR (NCS-Bande) und Massenspektrum charakterisiert. Es ist gut in organischen Lösungsmitteln, wie Methylenchlorid, löslich. Bei Raumtemperatur, insbesondere in wäßriger Umgebung, reagiert es langsam zu einem unlöslichen Oligomer ab. Bei Kühlung in geschlossenen Gefäß ist es stabil lagerbar.
2) Synthese von 1-(p-Maleinimidophenyl)-2,5,9,12-tetraazacyclotetradecan (R = Maleinimid) 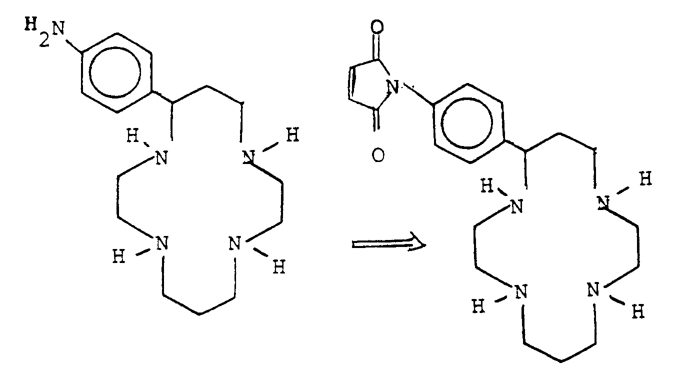 29 mg des Produkts von Beispiel 1c) werden in 1 ml wasserfreiem Gemisch 5:1 Aceton:Methanol teilweise gelöst und unter Feuchtigkeitsausschluß bei Raumtemperatur stark gerührt. Dazu werden 12 mg Maleinsäureanhydrid in 100 µl trockenem Aceton gegeben. Die Lösung wird zur Trockne eingeengt. Das Produkt wird mit 7 mg wasserfreiem Natriumacetat in 1 ml Acetanhydrid 2 h bei 50°C gerührt (Feuchtigkeitsausschluß) und dann wiederum eingeengt. Der Rückstand wird in 2 ml Wasser aufgenommen und 2 h bei 70°C gerührt. Beim Abkühlen fällt das Produkt aus. Es wird abfiltriert, mit Wasser gewaschen und lyophilisiert.
   Ausbeute 40 % d. Th. Charakterisierung über ¹H-NMR, Massenspektrum und IR-Spektrum.
3) Komplexierung von Tc-99m mit 1-(p-Aminophenyl)-2,5,9,12-tetraazacyclotetradecan (R = NH₂)
   In 0,5 ml 0,9 % Kochsalzlösung werden 5,1 mg des substituierten Cyclams gelöst. Von dieser Lösung nimmt man 0,1 ml (1 mg) ab, verdünnt mit 0,8 ml 0,9 % Kochsalzlösung, fügt 53 µg Sn²⁺ in Form von Zinn(II)-citrat hinzu und versetzt mit 0,1 ml (320 MBq)Tc-99m-Pertechnetat, das aus einem Mo-99/Tc-99m-Generator eluiert wurde. Die Reaktionslösung wird für 10 Minuten bei Raumtemperatur stehengelassen.
   Die radiochemische Reinheit wurde mit der Dünnschichtchromatographie und einem Filtrationstest untersucht.

### Dünnschichtchromatographie

Methode 1: Gelman ITLC SG / Methylethylketon oder Methanol:Wasser = 4:1
Methode 2: Whatman 1 / Methylethylketon

### Filtrationstest

Rund 1 ml der Lösung mit genau bekannter Aktivitätskonzentration wird über ein Filter (0,22 µm Porendurchmesser) filtriert und die Aktivitätskonzentration im Filtrat gemessen.

Die dünnschichtchromatographische Untersuchung zeigte keinerlei Pertechnetat an. Die gesamte Aktivität verblieb am Auftragspunkt. Die Anfärbung mit Ninhydrin zeigte, daß auch die inaktive Substanz einen R₁-Wert von 0 aufweist.

Um die Bildung von reduziertem, kolloidal anfallendem Technetium auszuschließen wurde der Filtrationstest durchgeführt. Im Filtrat wurden 98,2 % der eingesetzten Aktivität gefunden, so daß kein filtrierbares kolloidales Technetium vorgelegen hat.

Die analytische Untersuchung zeigt somit, daß der gewünschte Tc-99m-Komplex vollständig gebildet worden ist.

## Patentansprüche

1. Verfahren zur Markierung von Substanzen mit Technetium- und/oder Rheniumisotopen, dadurch gekennzeichnet, daß
a) die zu markierende Substanz mit einem makrocyclischen Komplexbildner der allgemeinen Formel I 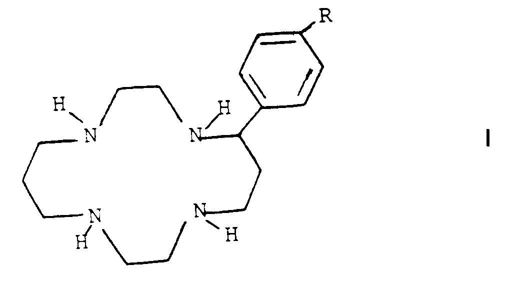 , worin
R 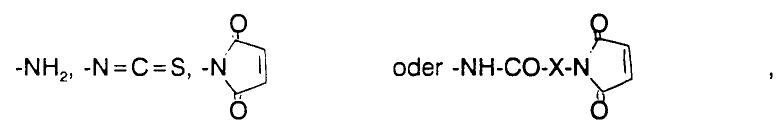 und
X (C₁-C₆)-Alkylen, (C₆-C₁₂)-Arylen, (C₁-C₆)-Alk-(C₆-C₁₂)-arylen bedeutet
umgesetzt wird und die so erhaltene Verbindung mit Tc-99, Tc-99m, Re-186 oder Re-188 markiert wird, indem man sie bei einem pH-Wert von unter 9 mit Pertechnetat-99, -99m oder Perrhenat-186, -188 und einem Reduktionsmittel versetzt oder daß man
b) zunächst den Tc-99-, Tc-99m-, Re-186- oder Re-188-Komplex durch Umsetzung einer Verbindung der Formel I mit der entsprechenden Per-Verbindung und einem Reduktionsmittel bei einem pH-Wert von unter 9 herstellt und anschließend diesen Komplex mit der zu markierenden Substanz umsetzt.

2. Verfahren nach Anspruch 1, dadurch gekennzeichnet, daß
X (C₁-C₄)-Alkylen, Phenylen, insbesondere 1,4-Phenylen oder (C₁-C₄)-Alk-1,4-phenylen bedeutet.

3. Verfahren nach den Ansprüchen 1 oder 2, dadurch gekennzeichnet, daß die Komplexierung in einem Ausmaß von größer als 90 %, bezogen auf eingesetztes Tc oder Re erfolgt.

4. Verfahren nach den Ansprüchen 1 bis 3, dadurch gekennzeichnet, daß die zu markierende Substanz zu der Klasse der Proteinen, Zucker, Fettsäuren, Hormonen, Polymeren, insbesondere zu den Antikörpern- bzw. deren Fragmenten zählen.

5. Verfahren nach Anspruch 4 dadurch gekennzeichnet, daß die zu markierende Substanz ein monoklonaler Antikörper bzw. dessen Fragment ist.

6. Verfahren nach den Ansprüchen 1 bis 5, dadurch gekennzeichnet, daß die zu markierende Substanz eine reaktive Gruppe enthält, die mit der reaktiven Gruppe R eine chemische Bindung ausbildet.

7. Verfahren nach den Ansprüchen 1 bis 6, dadurch gekennzeichnet, daß als Reduktionsmittel eine Sn-II-Verbindung, insbesondere ein komplexstabilisiertes Sn-II-Salz, verwendet wird.

8. Testkit zum Nachweis von Tumoren, bestehend aus zwei getrennten, lyophilisierten Komponenten, von denen eine einen Antikörper, der gegen Tumorassoziierte Antigene gerichtet ist, oder dessen Fragment im Gemisch mit einem Puffer enthält, wobei der Antikörper oder dessen Fragment mit der Verbindung gemäß Formel I verknüpft ist, und die andere ein Reduktionsmittel enthält, das zur Reduktion und Bindung des Technetiums oder Rheniums an der Antikörperkomponente benötigt wird.

## Patentansprüche (Patentansprüche für folgende(n) Vertragsstaat(en): ES)

1. Verfahren zur Markierung von Substanzen mit Technetium- und/oder Rheniumisotopen, dadurch gekennzeichnet, daß
a) die zu markierende Substanz mit einem makrocyclischen Komplexbildner der allgemeinen Formel I 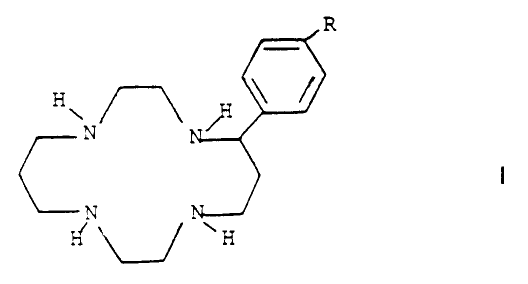 , worin
R 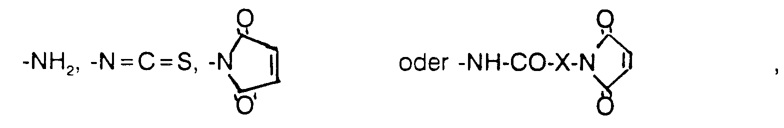 und
X (C₁-C₆)-Alkylen, (C₆-C₁₂)-Arylen, (C₁-C₆)-Alk-(C₆-C₁₂)-arylen bedeutet
umgesetzt wird und die so erhaltene Verbindung mit Tc-99, Tc-99m, Re-186 oder Re-188 markiert wird, indem man sie bei einem pH-Wert von unter 9 mit Pertechnetat-99, -99m oder Perrhenat-186, -188 und einem Reduktionsmittel versetzt oder daß man
b) zunächst den Tc-99-, Tc-99m-, Re-186- oder Re-188-Komplex durch Umsetzung einer Verbindung der Formel I mit der entsprechenden Per-Verbindung und einem Reduktionsmittel bei einem pH-Wert von unter 9 herstellt und anschließend diesen Komplex mit der zu markierenden Substanz umsetzt.

2. Verfahren nach Anspruch 1, dadurch gekennzeichnet, daß
X (C₁-C₄)-Alkylen, Phenylen, insbesondere 1,4-Phenylen oder (C₁C₄)-Alk-1,4-phenylen bedeutet.

3. Verfahren nach den Ansprüchen 1 oder 2, dadurch gekennzeichnet, daß die Komplexierung in einem Ausmaß von größer als 90 %, bezogen auf eingesetztes Tc oder Re erfolgt.

4. Verfahren nach den Ansprüchen 1 bis 3, dadurch gekennzeichnet, daß die zu markierende Substanz zu der Klasse der Proteinen, Zucker, Fettsäuren, Hormonen, Polymeren, insbesondere zu den Antikörpern- bzw. deren Fragmenten zählen.

5. Verfahren nach Anspruch 4, dadurch gekennzeichnet, daß die zu markierende Substanz ein monoklonaler Antikörper bzw. dessen -Fragment ist.

6. Verfahren nach den Ansprüchen 1 bis 5, dadurch gekennzeichnet, daß die zu markierende Substanz eine reaktive Gruppe enthält, die mit der reaktiven Gruppe R eine chemische Bindung ausbildet.

7. Verfahren nach den Ansprüchen 1 bis 6, dadurch gekennzeichnet, daß als Reduktionsmittel eine Zn-II-Verbindung, insbesondere ein komplexstabilisiertes Zn-II-Salz, verwendet wird.

8. Verfahren zum Nachweis von Tumoren, indem ein Testkit bestehend aus zwei getrennten, lyophilisierten Komponenten angewendet wird, von denen eine einen Antikörper, der gegen Tumor-assoziierte Antigene gerichtet ist, oder dessen Fragment im Gemisch mit einem Puffer enthält, wobei der Antikörper oder dessen Fragment mit der Verbindung gemäß Formel I verknüpft ist, und die andere ein Reduktionsmittel enthält, das zur Reduktion und Bindung des Technetiums oder Rheniums an der Antikörperkomponente benötigt wird.
